# EUROPÄISCHE PATENTANMELDUNG

(11) **EP 1 473 279 A1**
(43) Veröffentlichungstag der Anmeldung: **03.11.2004**
(21) Anmeldenummer: 04009505.1
(22) Anmeldetag: 22.04.2004
(51) Int. Cl.: C02F 3/28, C02F 11/04

(54) **Verfahren und anlage zur anaeroben verdauung von biomassen und erzeugung von biogas**

(30) Priorität: 30.04.2003 IT BZ20030024
(71) Anmelder: Ziegelei Gasser GmbH-Srl, 39040 Naz Sciaves (BZ) (IT)
(72) Erfinder: Mödinger, Fritz, 39040 Naz Sciaves (BZ) (IT); Binning, Rupert, 54341 Fell (DE); Gasser, Paul, 39040 Naz Sciaves (BZ) (IT)
(74) Vertreter: Oberosler, Ludwig

(57) **Zusammenfassung**

Verfahren und Anlage zur anaeroben Vergärung von Biomassen mit Erzeugung von Biogas und Schlamm welche eine Hydrolysierungsphase und eine folgende Fermentierung der Biomasse einschließen, wobei eine Hydrolysierungsphase (5) der Biomasse und mindestens zwei folgende Fermentationsphasen (7,8,9) umfasst werden und, sei es die Hydrolysierung (5), als auch die folgenden Fermentationen (7,8,9), in getrennten Behältern mit spezifischer Messung und Steuerung der Temperatur, der Durchmischung, des pH-Wertes und des Druckes erfolgen.

## Beschreibung

Die Erfindung betrifft ein Verfahren und eine Anlage zur anaerobischen Verdauung von Biomassen unterschiedlicher Herkunft, Konsistenz und chemischer Eigenschaften zwecks Produktion von Biogas.
Es sind Verfahren für die Produktion von Biogas mittels eines Fermenters oder Reaktors bekannt in welchem, unter Luftausschluss, bestimmte Biomassen anaerobisch verdaut werden, wobei mittels Mikroorganismen komplexe organische Substanzen (Lipide, Protide, Glucide) die in den Pflanzen oder in tierischen Restprodukten enthalten sind zerstört werden und es somit ermöglichen Energie mittels chemischer Reaktion unter Beihilfe von Enzymen, Pilzen und Mikroorganismen zu erhalten, wobei sich diese, bei jeweils bestimmten Verfahrensbedingungen, in der Biomasse im Substrat biologischer Herkunft (organische Masse) bilden. Die Fermentation erfolgt dank einer anaeroben Bakterienflora die sich in Abhängigkeit von der, im Fermenter herrschenden, Temperatur bildet. Diese Bakterien sind in der Biomasse enthalten und vermehren sich stark in einer geschlossenen Umgebung, wobei die Enzyme als Katalysator dienen; sie setzen die organische Substanz hauptsächlich in CH₄ (Methan) und CO₂ (Kohlendioxyd) um. Dieser Verdauungsablauf erfolgt wesentlich in drei Phasen:
- Hydrolyse der Cellulose, der Zucker und der Aminosäuren;
- saure Phase mit Bildung von einfachen organischen Säuren (z.B. Essigsäure) und Alkoholen (z.B. Ethylalkohol);
- Methanbildung mittels methanogener Bakterien durch Umsetzung von Säuren und Alkohole in Methan und Kohlendioxyd.

Diese bekannten Verfahren und die Anlagen für deren Verwirklung berücksichtigen nicht ausreichend, dass die anaerobische Bakterienflora von Bakterienstämmen gebildet ist welche sich optimal bei jeweils spezifischen Temperaturen zwischen 25°C und 45°C und bei spezifischen Umgebungsbedingungen entwickeln. Außerdem besteht das Risiko, dass sich während der Versäuerungsphase eine zu stark ausgebildete Versäuerung, bedingt durch die Bildung freier Säuren, auftritt und somit die Kontrolle des pH-Wertes schwierig ist. Dies bedingt, dass die Zeiten innerhalb welcher die einzelnen Verfahrensschritte ablaufen sich erhöhen, bzw. dass man Rückstände des Fermentationsprozesses erhält welche sich nicht für eine vorteilhafte Nutzung des Prozesses eignen da sie nicht umgesetzte oder nur teilweise umgesetzte Komponenten enthalten. Eine zu starke Versäuerung kann auch die Unterbrechung des biologischen Ablaufes und somit der Fermentation verursachen. Die vorgenannten Verfahren und die entsprechenden Anlagen berücksichtigen nicht ausreichend den Einfluss der Temperatur auf die Reaktionen und allgemein jene der Umgebungsverhältnisse unter welchen die Abbaureaktionen ablaufen. Insbesondere während der Phase der Säurebildung können die Bedingungen für eine zu hohe Versäuerung auftreten. Die zu hohe Bildung freier Säuren und die somit schwierige Kontrolle des pH-Wertes bewirkt eine Verlangsamung des Ablaufs der Reaktion und eine unvollständige Umwandlung des Substrates mit folgender Erhaltung von Restprodukten welche für eine folgende Verwertung, wegen dem hohen Anteil von organischen Substanzen, ungeeignet sind.

Die Erfindung stellt sich die Aufgabe ein geeignetes Verfahren zur anaeroben Digestion von Biomassen der oben erwähnten Art sowie einer dafür geeigneten Anlage zu verwirklichen, mittels welcher die Umwandlung der Biomasse, des Substrats biologischer Herkunft (organische Masse) zwecks Verarbeitung biologische Abfälle verschiedener Herkunft, verschiedener Stückgröße und Konsistenz und mit unterschiedlichem C/N Verhältnis und unterschiedlicher Feuchte, bei gleichzeitiger Optimierung der Dauer des Fermentations-, bzw. Digestionsprozesses, bei gleichzeitiger Steigerung des Umwandlungsgrades des methanogenen Substrates, zu ermöglichen um so feste und flüssige Rückstände zu erhalten die als Bestandteil in anderen oder im gleichen Produktionsprozess (Recycling) oder in verschiedenen Produktionszyklen oder in Recyclingverfahren, eingesetzt werden können.
Die Erfindung stellt sich weiters die Aufgabe folgende Vorteile zu erreichen:
- höhere Ausbeute an Biogas,
- Erhalt fester und flüssiger Restmengen welche für den Einsatz als Zusätze in Produktionsprozessen geeignet sind.

Zur Lösung dieser Aufgabe schlägt die Erfindung ein Verfahren vor welches in folgender Weise gegliedert ist:
- Hydrolyse der Polysacharide, der Proteine, der Lipide;
- Versäuerung unter Bildung einfacher biogener Säuren;
- ein- oder mehrstufige Methanisierung;
um so durch die Trennung der Versäuerungsstufe von der methanogenen Stufe eine leichtere Kontrolle des pH-Wertes zu erreichen und durch Vorsehen einer Kaskadenfermentierung zwecks Schaffung der jeweils die idealen Verhältnisse für die spezifischen Anforderungen der einzelnen Bakterienstämme der anaeroben Bakterienflora zu schaffen.

Um dies zu erreichen sieht die Erfindung ein Verfahren vor welches wie folgt gegliedert ist:
- eine spezifische Vorbehandlung der diversen Biomassen, oder von Teilen dieser, mit mechanischer Zerkleinerung und/oder einer Dampfbehandlung und/oder durch Erwärmung auf Temperaturen welche eine "Hygienisierung" der Biomassen bewirkt;
- eine Kaskadenfermentation um die idealen Bedingungen für die jeweils spezifischen Anforderungen der einzelnen Stämme der Bakterienflora, insbesondere um eine Methanisierung während mehrerer Stadien und getrennt von der Versäuerungsphase, zu schaffen;
- das Rückführen eines Teiles des Schlammes, welcher im letzten Stadium des Verfahrens produziert wird, in die Phasen der Hydrolyse und/oder der Fermentation zwecks Zuführung von Enzymen welche als biologische Katalysatoren wirken;
- das Eindicken des Schlammes mittels eines Hydrozyklons durch Abscheidung der Restflüssigkeiten.

Die erfindungsgemäße Anlage besteht aus:
- einer Schneideanlage für die Zerkleinerung der Biomassen, welche insbesondere für die Biomassen mit einem C/N-Verhältnis höher als 30 und einem Feuchtigkeitsgehalt unter 30% eingesetzt wird;
- einem Fermenter welcher aus getrennten und unterschiedlichen Reaktoren, zwecks einer wirksameren Kontrolle der Reaktionsbedingungen der einzelnen Verfahrensphasen, besteht;
- einer Wärmebehandlung mit Einführung von heißem Wasserdampf direkt in den Strom der Biomasse und/oder mittels Wärmeaustauscher um die thermochemische Umwandlung und eventuell um die "Hygienisierung" bestimmter Biomassen (z.B. stärkehaltiger Biomassen) zu erhalten;
- einem Wärmetauscher um die Temperatur der Biomasse am Auslauf nach dem Hydrolyseprozess auf den idealen Wert für den folgenden Versäuerungsprozess zu bringen; die gewonnene Wärmeenergie kann für das Vorwärmen oder für die Wärmezufuhr in einer oder in mehreren Verfahrensphasen genutzt werden.

Die verschiedenen Vorrichtungen der Anlage sind mit Wärmefühlern und Mischern ausgerüstet um die optimalen thermischen Bedingungen für die jeweils optimale Wirkung der spezifischen Bakterienstämme zu erzielen.

Die Erfindung wird anhand der beigefügten Zeichnungen welche schematisch den erfindungsgemäßen Verfahrensablauf, bzw. die erfindungsgemäße Anlage, zur anaerobischen Digestion von Biomassen unterschiedlicher Herkunft, Konsistenz und chemischer Eigenschaften, für die Erzeugung von Biogas, eingedicktem Schlamm und flüssigen Reststoffen, darstellen.

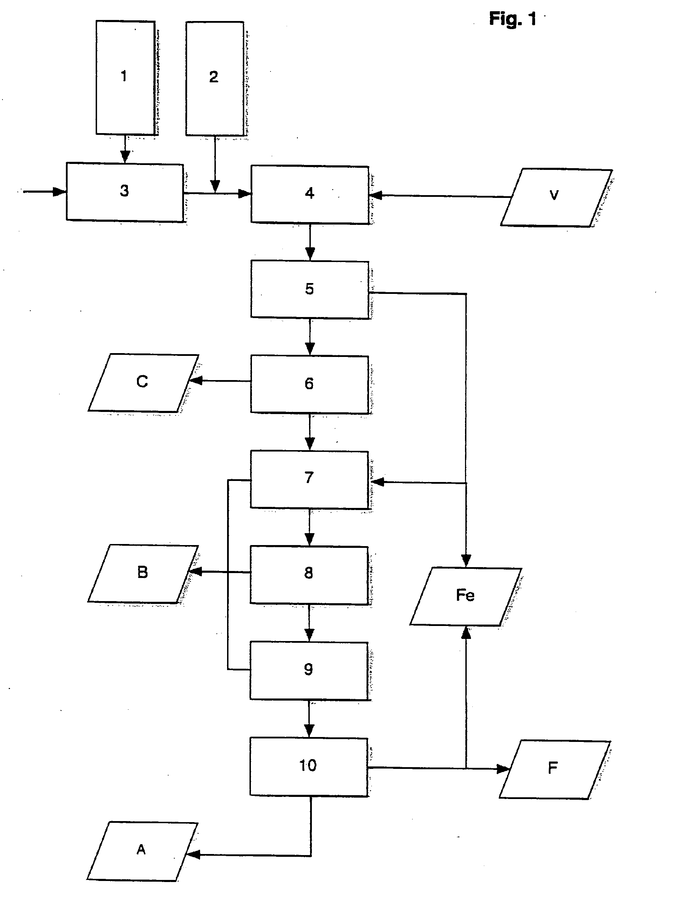

Die Fig. 1 zeigt ein Blockdiagramm des erfindungsgemäßen Verfahrens.

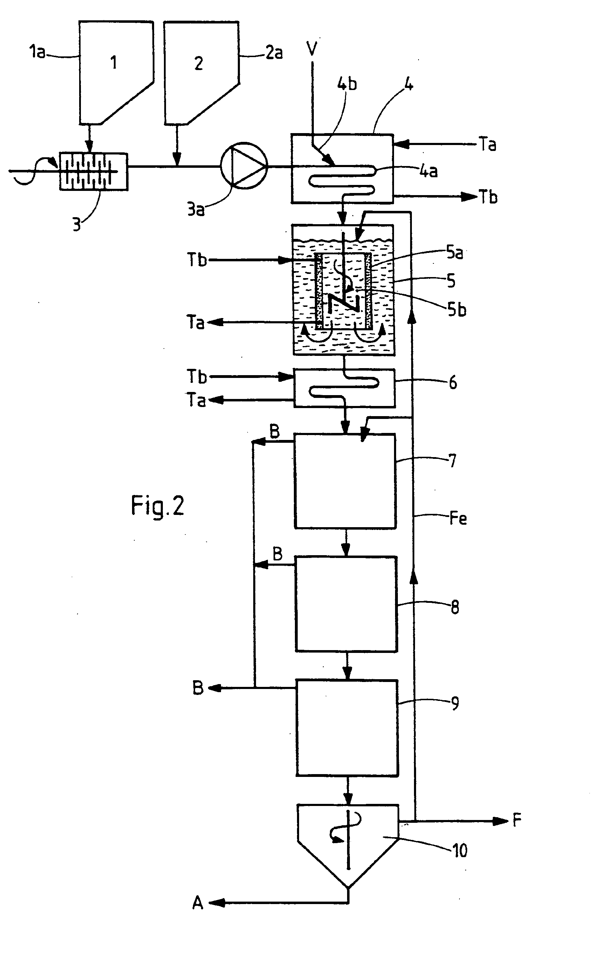

Die Fig. 2 zeigt ein Blockdiagramm der erfindungsgemäßen Anlage welche nach dem in Fig. 1 dargestellten Verfahren arbeitet.

Von den Lagern oder Silos 1a, 2a welche Bioabfälle 1 vom Typus grober Stückgröße mit einem C/N Verhältnis größer als ca. 30 und einem Feuchtigkeitsgehalt unter 30% und organische Schlämme 2 und/oder Biomasse feiner Stückgröße mit einem C/N Verhältnis vorteilhafterweise unter 30 und einem Feuchtigkeitsgehalt vorteilhafterweise über 30% enthalten, wird die Mischung 1, 2 entnommen. Diese Lademenge wird der Schneidanlage 3, zwecks Zerkleinerung in kleinere Stückgröße, zugeführt und mittels Pumpe 3a einem Vorwärmer zugeleitet wo di Biomasse einer Wärmezufuhr (Ta - Tb) mittels Wärmetauscher 4a unterzogen wird. Wenn erforderlich kann dazu Wasserdampf V eingesetzt werden welcher am Einlaufbereich, am Auslaufbereich des Wärmetauschers in die Biomasse oder im Wärmetauscher 4 selbst eingedüst werden kann.

Die vorgenannten Wärmebehandlungen können für die Hygienisierung der Biomasse und/oder für die thermische Spaltung der Stärken erforderlich sein. Üblicherweise bewirkt die Vorwärmung in der Biomasse eine Temperatur welche nicht die 100°C übersteigt; anschließend wird die Biomasse dem Hydrolisierer zugeführt welcher, wie auch die folgenden Fermenter 7, 8, 9, innen mit einem vertikal stehenden zweiwandigen Rohr 5a ausgestattet ist, innerhalb welchem ein Kühlmedium (Tb - Ta), bzw. ein Wärmemedium, zwecks Kontrolle der Temperatur, zirkuliert.

Die erfindungsgemäße Hydrolyse findet bei Temperaturen zwischen 40°C - 100°C statt um die organischen Polymersubstrate wie komplexe Stärken, Pectin Hemicellulosen, durch die Wikung von Enzymen (Amilasen, Pektinasen, Hemicellulasen) zu spalten wodurch sich vorwiegend Monosacharide und ein Substrat niedriger Viskosität bildet welches für die nachfolgende Aktivität der methanogenen Bakterien besser zur Verfügung steht. Die Hydrolysestufe ist allgemein (in bekannten Anlagen) zur Erzeugung von Biogas vorgesehen, diese erfolgt jedoch bei beachtlich niedrigeren Temperaturen und ohne Kontrolle der Temperatur und der Durchmischung in Abhängigkeit von den Eigenschaften und der Konzentration der Biomasse. Erfindungsgemäß hingegen können während der Hydrolyse der Biomasse Enzyme (Amilasen, Pektinasen, Hemicellulasen) oder andere Stoffe wie z.B. einige Fermente beigesetzt werden, wobei eventuell auch eine teilweise kontrollierte Rückführung Fe der Schlämme F, welche am Ende des Verfahrensablaufes entnommen werden, vorgesehen ist.

Vor der Zuführung des Substrates in die Fermenter 7, 8, 9 ist es angebracht dieses in einem Wärmetauscher zu kühlen, wobei gegebenenfalls die an das Kühlmedium Tb - Ta abgegebene Wärme für die Erwärmung oder Vorwärmung der Vorrichtungen oder Teile der selben Anlage oder einer damit verbundenen Anlage genutzt werden kann.

Erfindungsgemäß erfolgt die Fermentation kaskadenartig in getrennten Fermentern 7, 8, 9 die jeweils einzeln steuerbar sind, in denen spezifische Kulturen gezielt, unter optimalen Bedingungen betreffend die Temperatur, die Durchmischung, den Gasabzug und den pH-Wert, wirken.

Es ist bekannt, dass die verschiednen anaerobischen Bakterienstämme ihre optimale Aktivität jeweils bei unterschiedlichen Temperaturen entwickeln: die Psicrophilen bei Temperaturen unter 15°C, die Mesophilen bei Temperaturen zwischen 25°C und 45°C und die Thermophilen bei Temperaturen über 45°C.

Im ersten Fermenter 7 erfolgt, immer unter spezifischer Überwachung der Temperatur, des pH-Wertes, der Durchmischung und der Gasentnahme, die Bildung der Säuren und/oder die Versäuerung durch Wirkung der Säurebakterien. Die hauptsächlichen Kulturstämme sind vom Typ Lactobazillus (z.B. LB casei, LB plantarum) und vom Typ Streptococcus (z.B. SC lactii, SC cemoris).

In den nachfolgenden Fermentern 8, 9 wirken die eigentlichen methanogenen Bakterien welche die kontrollierte Zufuhr von biogenen Säuren im, aus dem Fermenter 7 kommenden, Substrat verlangen. In diesen Fermentern wirken gemischte Kulturen, vorwiegend vom Typ Methanobacterium, Methanosarcina und Methanococcus. In Symbiose mit den methanogenen Bakterien agieren Cellulitisbakterien welche die Hydrolyse und die Verdauung der organischen Stoffe mit niederer Löslichkeit, wie z.B. die Zellulose, bewirken.

Da sich diese anaeroben Bakterien nur sehr langsam vermehren, weil ein anaerober Metabolismus eine geringe Energiedichte aufweist, ist es notwendig eine teilweise Rückführung Fe des am Ende des Verfahrens anfallenden Schlammes F in die ersten Phasen der Fermentation 5, 7 vorzusehen.

Die methanogenen Bakterien sind in beinahe allen Stoffen die einem anaeroben Abbau unterliegen (Klärschlamm, Moorschlämme, Bergbauschlämme) und auch in Symbiose im Magen der Wiederkäuer vorhanden, weshalb die Ausscheidungen dieser sich als leicht zur Verfügung stehende Startkulturen eignen.

Aus jedem Fermenter wird in kontrollierter Weise das Biogas B abgezogen welches gegebenenfalls als erneuerbarer Energieträger zur Verfügung steht.
Aus dem letzten Fermenter 9 wird das vollkommen abgebaute Substrat in einen Hydrozyklon 10 geleitet wo die Abscheidung der Restflüssigkeit A erfolgt, diese Komponenten sind in verschiedenen Produktionszyklen verwertbar und zwar, z.B. was den festen Teil betrifft, als Porosierungsmittel oder was den flüssigen Teil betrifft, als Prozesswasser.

Das erfindungsgemäße Verfahren und die entsprechende Anlage ermöglichen die Verarbeitung von Biomasse sehr unterschiedlicher Herkunft, Beschaffenheit und chemischer Eigenschaften, wobei durch den vollständigen Abbau der organischen Substanz, die Probleme der Entsorgung der flüssigen Reststoffe und der eingedickten Schlämme gelöst werden und das erzeugte Biogas, sowie die gewonnene Wärme, für den Betrieb der Anlage selbst, bzw. als erneuerbare Energie, z.B. für den Betrieb von andersartigen Anlagen genutzt wird.

Natürlich sehen alle Phasen des Verfahrens die Aufzeichnung der Daten betreffend Temperatur, Druck, Flussgeschwindigkeit und chemische Zusammensetzung, zwecks spezifischer Prozesssteuerung, vor welche es ermöglicht einen hohen Wirkungsgrad betreffend die Erzeugung von Biogas, von Schlämmen F und von Restflüssigkeiten A, welche problemlos in verschiedenen Herstellungsverfahren verwendbar sind, zu erreichen.

## Patentansprüche

1. Verfahren zur anaeroben Digestion von Biomassen mit Erzeugung von Biogas und Schlamm welches eine Hydrolysierungsphase und eine folgende Fermentierung der Biomasse einschließt, **dadurch gekennzeichnet, dass** es eine Hydrolysierungsphase (5) der Biomasse und mindestens zwei folgende Fermentationsphasen (7, 8, 9) umfasst und dass, sei es die Hydrolysierung (5) als auch die folgenden Fermentationen (7, 8, 9), in getrennten Behältnissen mit spezifischer Messung und Steuerung der Temperatur, der Durchmischung, des pH-Wertes und des Druckes erfolgen.

2. Verfahren nach Anspruch 1, **dadurch gekennzeichnet, dass** die Hydrolysierungsphase (5) der Biomasse bei einer Temperatur zwischen 40°C und 100°C und anschließend an eine Vorwärmungsphase (4) des Substrates mittels Wärmetauscher (4a) und/oder mittels Eindüsung von Wasserdampf (V) erfolgt und dass der Hydrolysierungsphase eine Kühlphase (6) folgt.

3. Verfahren nach Anspruch 1, **dadurch gekennzeichnet, dass** in der Hydrolysephase (5) der Biomasse und/oder in einer oder in mehreren Fermentationsphasen (7, 8, 9) eine kontrollierte Zufuhr von Säureenzymhaltigen Schlämmen (Fe), die am Verfahrensende anfallen, erfolgt.

4. Verfahren nach Anspruch 1, **dadurch gekennzeichnet, dass** nach der Fermentation (7) welche durch säurebildende Enzyme erfolgt, mindestens eine Fermentationsphase (8, 9) unter Einwirkung von spezifischen methanogenen Bakterien vorgesehen ist, wobei diese gegebenenfalls in den verschiedenen Fermentern in welchen spezifische Bedingungen betreffend Temperatur-, pH-Wert, Durchmischung und Druck herrschen, von unterschiedlichem Typ sein können.

5. Verfahren nach Anspruch 1, **dadurch gekennzeichnet, dass** die Schlämme (F) welche am Ausgang des letzten Fermenters (9) anfallen, in einem Hydrozyklon (10) oder mittels Zentrifuge, unter Abscheidung der Restflüssigkeit (A), eingedickt werden.

6. Verfahren nach Anspruch 1, **dadurch gekennzeichnet, dass** die Wärme welche während der Kühlung (6) abgeführt wird, für die Vorwärmung oder Heizung in einer oder in mehreren, Wärmezuführung verlangenden, Phasen (4, 5, 7, 8, 9) des Verfahrens selbst oder in einem anderen verbundenen Verfahren, genutzt wird.

7. Verfahren nach Anspruch 1, **dadurch gekennzeichnet, dass** das während der unterschiedlichen Fermentationsphasen (7, 8, 9) produzierte Biogas (B) kontrolliert von jedem einzelnen Fermenter abgezogen wird.

8. Verfahren nach Anspruch 1, **dadurch gekennzeichnet, dass** für die anaerobe Digestion des Substrates ein Hydrolysator (5) vorgesehen ist welcher in Reihe mit mindestens zwei Fermentern (7, 8, 9) verbunden ist und dass, sei es der Hydrolysator (5) als auch die Fermenter mit Wärmetauscher (5a), mit mechanischem-, hydraulischem- oder Gas-Mischer (5b) und mit Sonden oder Fühlern für die Ermittlung der Werte betreffend die Temperatur, den pH-Wertes, den Druckes, den Durchmischungsgrades ausgestattet sind und dass der Wärmetauscher (5a), der Mischer (5b), die Entnahme von Biogas (B) und die Einführung der rückgeführten Schlämme (Fe), abhängig von den besagten ermittelten Werten und der spezifischen Anforderungen, der im Hydrolysator (5) und in den verschiednen Fermentern (7, 8, 9) wirkenden Bakterienflora, betrieben werden.

9. Anlage nach Anspruch 8, **dadurch gekennzeichnet, dass** vor der Hydrolyse (5) ein Vorwärmer (4) mit Wärmetauscher (4a) und eine Vorrichtung für die Eindüsung (4b) von Wasserdampf (V) vor dem Wärmetauscher (4a), nach diesem oder in einer Zwischenposition, vorgesehen ist.

10. Anlage nach Anspruch 8, **dadurch gekennzeichnet, dass** nach dem Hydrolysator (5) ein Wärmetauscher (6) für die Kühlung des Substrates vorgesehen ist.

11. Anlage nach Anspruch 8, **dadurch gekennzeichnet, dass** eine kontrollierte Rückführung (Fe) der Schlämme welche am Ende der Anlage ausscheiden, durch Einführung in den Hydrolysator (5) und/oder in einen oder in mehrere Fermenter (7, 8, 9), erfolgt.
